# EUROPÄISCHE PATENTSCHRIFT

(11) **EP 2 217 262 B1**
(45) Veröffentlichungstag und Bekanntmachung des Hinweises auf die Patenterteilung: **08.01.2014**
(21) Anmeldenummer: 08860644.7
(22) Anmeldetag: 28.11.2008
(51) Int. Cl.: A61K 38/17, A61P 31/04, C07K 14/47

(54) **C-TERMINALE IFAPSORIASINFRAGMENTE ALS ANTIMIKROBIELLE PEPTIDE UND DEREN VERWENDUNG IN DER BEHANDLUNG VON PSEUDOMONAS INFEKTIONEN**
C-TERMINAL IFAPSORIASIN FRAGMENTS AS ANTIMICROBIAL PEPTIDES FOR USE IN THE TREATMENT OF PSEUDOMONAS INFECTIONS
FRAGMENTS D'IFAPSORIASINE À TERMINAISON C EN TANT QUE PEPTIDES ANTIMICROBIENS ET LEUR UTILISATION DANS LE TRAITMENT DES INFECTIONS DE PSEUDOMONAS

(30) Priorität: 12.12.2007 DE 102007059891
(43) Veröffentlichungstag der Anmeldung: 18.08.2010
(73) Patentinhaber: Universitätsklinikum Schleswig-Holstein, 24105 Kiel (DE)
(72) Erfinder: SCHRÖDER, Jens-Michael, 24241 Blumenthal (DE); HANSMANN, Britta, 24106 Kiel (DE); WU, Zihong, 24119 Kronshagen (DE)
(74) Vertreter: Müller Fottner Steinecke
(86) Internationale Anmeldenummer: PCT/DE2008/001984
(87) Internationale Veröffentlichungsnummer: WO 2009/074133

(56) Entgegenhaltungen:
- HANSMANN B ET AL: "Human ifapsoriasin, a novel member of the ' S100 fused type protein ' family, is expressed in healthy skin" JOURNAL OF INVESTIGATIVE DERMATOLOGY, Bd. 127, Nr. Suppl. 2, Oktober 2007 (2007-10), Seite S61, XP002561186 37TH ANNUAL MEETING OF THE EUROPEAN-SOCIETY-FOR-DERMATOLOGICAL-RESEA RCH; ZURICH, SWITZERLAND; SEPTEMBER 05 -08, 2007 ISSN: 0022-202X
- WU Z H ET AL: "Identification of ifapsoriasin as a novel and putative new member of the " S100 fused type protein" family" JOURNAL OF INVESTIGATIVE DERMATOLOGY, Bd. 126, Nr. Suppl. 3, August 2006 (2006-08), Seite 13, XP002561187 & 36TH ANNUAL MEETING OF THE EUROPEAN-SOCIETY-OF-DERMATOLOGY-RESEARCH (ESDR); PARIS, FRANCE; SEPTEMBER 06 -07, 2006 ISSN: 0022-202X
- TOULZA E ET AL: "Large-scale identification of human genes implicated in epidermal barrier function" GENOME BIOLOGY 20070611 GB, Bd. 8, Nr. 6, 11. Juni 2007 (2007-06-11), XP002561188
- HAUSMANN B ET AL: "A conserved C-terminal fragment of lfapsoriasin is a selectively Pseudomonas ssp.-killing antimicrobial peptide" JOURNAL OF INVESTIGATIVE DERMATOLOGY, Bd. 128, Nr. Suppl. 1, April 2008 (2008-04), Seite S182, XP002561189 & INTERNATIONAL INVESTIGATIVE DERMATOLOGY MEETING; KYOTO, JAPAN; MAY 14 17, 2008 ISSN: 0022-202X

## Beschreibung

Die Erfindung betrifft die Verwendung eines Peptids mit der in SEQ ID:NO 1, SEQ ID:NO 2 oder SEQ ID:NO 3 dargestellten Sequenz zur Herstellung eines antimikrobiell wirkenden Arzneimittels zur Behandlung von Infektionen durch Bakterien der Gattung *Pseudomonas*.

Die Körperoberflächen mehrzelliger Lebewesen stellen eine wichtige Grenzschicht gegenüber der Umgebung dar und liefern eine erste Verteidigungslinie gegenüber eindringenden Mikroorganismen. Insbesondere antimikrobiell wirksame Peptide, die zahlreich in der Epidermis und vor allem in der darüber liegenden Hornschicht vorhanden sind, nehmen an dem Verteidigungssystem teil. Sie kontrollieren mikrobielles Wachstum in den ersten Stunden nach einer oberflächlichen Verletzung und während der Wundheilung. Insbesondere sind sie bei einigen Hauterkrankungen zu finden.

In der Haut des Menschen sind bisher sechs Klassen antimikrobieller Peptide entdeckt worden, die ß-Defensine, RNase-7, Psoriasin (S100-A7), Cathelicidin hCAP18, Dermcidin und Lysozym. RNase 7, Lysozym und Dermcidin werden konstitutiv in gesunder Haut gebildet, während die ß-Defensine und das Cathelicidin nur durch entzündliche Stimuli in humanen Keratinozyten induziert werden und damit in erster Linie als Reaktion auf Verletzungen wirken.

Defensine sind kleine kationische und amphipatische Peptide mit einem Molekulargewicht von 3 bis 5 kDa, sie haben eine antibakterielle und eine antimykotische Wirkung. Während die α-Defensine HNP 1-4 beispielsweise in humanen neutrophilen Granulozyten, die sich in entzündetem Gewebe anreichern, gebildet werden, findet man die ß-Defensine hBD-2 und hBD-3, die von Epithelzellen produziert werden, in der entzündeten Haut und anderen entzündeten Körperoberflächen.

RNase 7 ist ein kleines kationisches Protein, das von Epithelzellen insbesondere der Haut konstitutiv produziert und bereitgestellt wird. Es zeigt ein breites antimikrobielles Wirkungsspektrum mit besonders hoher Wirksamkeit gegenüber Gram-positiven Darmbakterien wie beispielsweise Enterokokken.

Psoriasin ist ebenfalls ein kleines Protein, das von Hautkeratinozyten produziert wird. Es ist ein gegen das Darmbakterium *Escherichia coli* spezifisch wirksames antibiotisches Protein, das auf der Hautoberfläche in antibiotisch wirksamen Konzentrationen nachweisbar ist.

Dermcidin ist ein weiteres antibiotisches Peptid, das spezifisch von Zellen der Schweißdrüsen produziert wird und gegen Staphylokokken und *Escherichia coli* wirksam ist. Es ist im Schweiß nachweisbar und wirkt hier als Schutzfaktor vor Staphylokokken-Infektionen.

Allgemein sind antimikrobielle Peptide endogene, Gen-kodierte Peptide mit besonderer Bedeutung für die frühe Phase der Abwehr gegen mikrobielle Erreger, inklusive Infektionsprophylaxe. Einige sind permanent als antimikrobiell aktive Peptide vorhanden, weitere, beispielsweise das Peptid LL-37, werden erst nach Abspaltung größerer, per se antibiotisch inaktiver Proteine gebildet und wiederum andere werden lokal neu synthetisiert. Sie können deshalb, je nach Typus, innerhalb von Minuten bis Stunden nach dem ersten Kontakt mit dem Pathogen nachgewiesen werden.

Allerdings wirken die bekannten antimikrobiellen Peptide nicht gegen alle mikrobiellen Pathogene in gleicher Weise. Dermcidin wirkt beispielsweise nicht bei Infektionen mit *Pseudomonas aeruginosa*, einer wichtigen Ursache für Hautinfektionen insbesondere bei Brandverletzungen und für Lungeninfektionen, insbesondere bei der Mukoviszidose.

Für die Bekämpfung pathogener Mikroorganismen werden präventiv oder kurativ auch Antibiotika eingesetzt, also Stoffe mikrobiellen Ursprungs, die andere Mikroorganismen an ihrem Wachstum hemmen oder sogar abtöten. Im Gegensatz zu den oben erwähnten antimikrobiellen Peptiden haben Antibiotika in der Regel eine selektive Wirkung. Viele Mikroorganismen besitzen eine natürliche Unempfindlichkeit gegenüber einem Antibiotikum, sie können diese sogenannte Antibiotika-Resistenz aber auch im Verlauf des Wachstums in Gegenwart von Antibiotika entwickeln.

Durch Mutations- und Selektionsprozesse sowie durch die Ausbildung von Resistenzen ergeben sich nicht nur im Klinikalltag sondern auch bei der Arzneimittel- und Kosmetikalierstellung immer häufiger Probleme mit mikrobiellen Pathogenen, die nicht oder nicht effektiv bekämpft werden können.

Vor diesem Hintergrund besteht ein ständiger Bedarf an neuen antimikrobiell wirkenden Agenzien, die präventiv oder kurativ eingesetzt werden können.

Damit liegen der vorliegenden Offenbarung zugrunde, weitere antimikrobiell wirkende Peptide bereitzustellen, die als gut zugängliches Arzneimittel mit biologischer und therapeutischer Aktivität eines natürlichen Stoffes verwendet werden können sowie einen Weg zu ihrer Produktion aufzuzeigen.

Erfindungsgemäß wird diese Aufgabe durch antimikrobiell wirkende Peptide mit den in Ansprüchen 1 bis 10 aufgeführten Merkmalen gelöst.

Die Erfindung wird in den Fig. 1 bis 5 näher veranschaulicht. Es zeigen:
- Fig. 1: Nucleotid- und Aminosäuresequenz der humanen Ifapsoriasin cDNA,
- Fig. 2: eine schematische Strukturdarstellung des humanen Ifapsoriasin-Proteins,
- Fig. 3: die Isolierung des IFPS₂₂₄₄₋₂₃₉₁-SUMO-Fusionsproteins mittels Umkehrphasen-HPLC,
- Fig. 4: Umkehrphasen-HPLC des IFPS₂₂₄₄₋₂₃₉₁-SUMO-Fusionsprotein-Verdaus und Isolierung von IFPS₂₂₄₄₋₂₃₉₁,
- Fig. 5: MIC (minimale Hemmhofkonzentration) für das IFPS-Peptid 2 (SEQ ID:NO 2), und
- Fig. 6: LD₉₀ (Letale Dosis 90, Konzentration bei der 90% der Mikroorganismen abgetötet werden) für das IFPS-Peptid 2 (SEQ ID:NO 2).

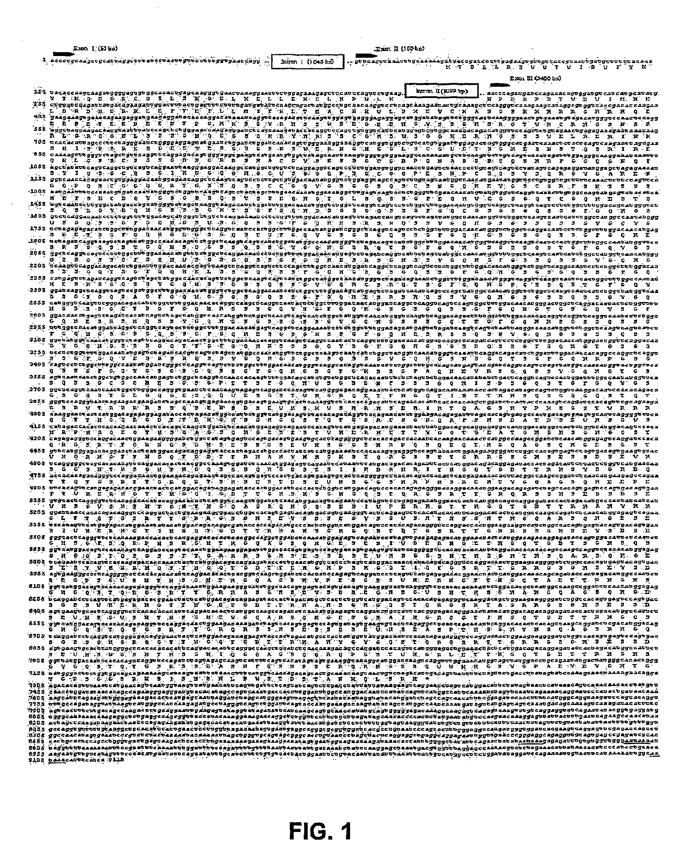

Für das oben beschriebene Ifapsoriasin (im Folgenden: IFPS) wurde die in Fig. 1 dargestellte kodierende Nukleinsäuresequenz (cDNA) gefunden. Die komplette Nucleotidsequenz der humanen Ifapsoriasin-cDNA (9117 bp) wurde durch Klonierung und Sequenzierung bestätigt. Die abgeleitete Aminosäuresequenz des offenen Leserahmens wurde direkt unter den kodierenden Basen dargestellt. Die unterstrichen fett dargestellten Nucleotide geben die kanonische Poly(A)-Signalsequenz an. Das IFPS-Gen besteht aus drei Exons und zwei Introns. Die Exon/Intron-Grenzen wurden mithilfe des BLAST Search-Programms ermittelt. Die cDNA-Sequenzdaten sind unter der GenBank beim "National Center for Biotechnology Information" (NCBI) unter Zugangsnummer AY827490 verfügbar. Durch die Analyse der kodierenden Nucleotidsequenz wurde das Gen des neuen Proteins IFPS auf dem Chromosom 1q21 lokalisiert. IFPS besitzt ein sehr restriktives Expressionsmuster, da es nur in humaner Haut und Vaginalschleimhaut gebildet wird.

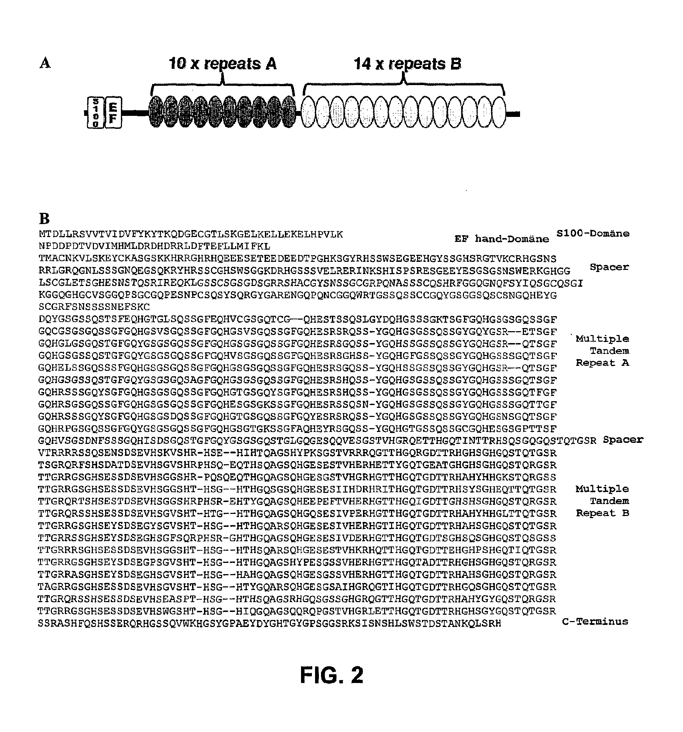

Das IFPS-Gen kodiert ein 2391 Aminosäuren umfassendes Protein, das aus einem sogenannten S100-Teil, einem sogenannten EF-Hand-Teil, einen Spacer, 10 Repeat-Domänen A, 14 Repeat-Domänen B und einem C-Terminus besteht (Fig. 2).

Das 248 kDa IFPS Polyprotein wird in der Haut proteolytisch zu verschiedenen Peptiden prozessiert. Mit zwei unterschiedlichen antimikrobiellen Assays konnten die Erfinder zeigen, dass C-terminale Peptidfragmente dosisabhängig und in selektiver Weise toxisch sind für Feuchtkeime, insbesondere *Pseudomonas aeruginosa*, *Pseudomonas stutzeri* und *Pseudomonas syringae.*

Die vorliegende Erfindung stellt des Weiteren ein Herstellungsverfahren für die Peptide sowie die Verwendung der Peptide als Arzneimittel für verschiedene therapeutische und diagnostische Indikationen bereit. Dazu können die Peptide als hochreine Stoffe oder - wenn für die Verwendung ausreichend - innerhalb eines teilweise aufgereinigten Peptidgemisches oder als Gemisch mehrerer Peptide oder auch ihre Gensonden verwandt werden.

Vor diesem Hintergrund betrifft die Erfindung ferner Nukleinsäuremoleküle mit einem für ein Peptid kodierenden Sequenzabschnitt, einen Expressionsvektor mit einem derartigen Nukleinsäuremolekül sowie gegebenenfalls Kontrollsequenzen, insbesondere für Replikation, Transkription und/oder Translation, sowie eine Wirtszelle, die mit dem Expressionsvektor transfiziert oder transformiert ist. Hierfür stehen verschiedene Expressionsvektoren routinemäßig zur sekretorischen oder direkten cytoplasmatischen Expression zur Verfügung.

Nachdem die Aminosäuresequenz der Peptide bekannt ist, kann mithilfe des genetischen Codes eine entsprechende Nukleinsäuresequenz abgeleitet werden, wobei optimierte Codons für unterschiedliche Wirte (Bakterien, Hefe, Säugerzellen, Pflanzen) verwendet werden können. Bevorzugt ist jedoch die sich aus Fig. 1 ergebende Codonwahl.

Wenn Peptide als Fusionsproteine synthetisiert werden, kann die Herstellung und Reinigung der Peptide erleichtert werden. Für Aminosäureabschnitte oder Domänen bekannter Proteine kodierende Sequenzen werden dabei an für die Peptid-de kodierenden Nukleinsäuren anfusioniert, so dass bei der Expression ein durchgehendes Protein erzeugt wird. Beispiele für solche anfusionierten Aminosäureabschnitte sind die Histidin-"Tags", durch die exprimierte Fusionsproteine über Nickel-Chelat-Säulen gereinigt werden können, oder antigene Determinanten, die es erlauben, die Peptide über geeignete Antikörperaffinitätssäulen zu reinigen.

In einem Ausführungsbeispiel ist es bevorzugt, wenn das Peptid mit einem weiteren Peptid oder Protein zu einem Fusionsprotein verbunden ist, wobei ein SUMO-Protein, ein Histidin-"Tag", ein proteolytischer Spalt-"Tag" und das IFPS-Peptid, vorzugsweise in der angegebenen Reihenfolge, verwendet wird.

Da IFPS natürlicherweise vermehrt in feuchten Haut- und Schleimhautbereichen gefunden wird, eignen sich die Peptide besonders gut zur Behandlung von Erkrankungen, die bei Organbesiedlung durch Feuchtkeime, besonders durch *Pseudomonas aeruginosa* entstehen.

Aufgrund der biologischen Wirkung und des natürlichen Vorkommens der IFPS-Peptide ist gezeigt, dass die Präparate als Mittel zur Therapie von infektiösen Erkrankungen vieler Epithelorgane und zur Infektionsprophylaxe auf Grenzflächen, insbesondere der Haut, des Nasen-Rachenraumes, der Augen, der Atemwege, des Magen-Darm-Traktes und des Urogenitaltraktes anwendbar sind.

Die Peptide können auch eingesetzt werden zur Behandlung von Systemerkrankungen bei Mangel dieser Peptide zur Behandlung von Erkrankungen des menschlichen Organismus, insbesondere mit Beteiligung der Atemwege, des Magen-Darm-Traktes und des Urogenitaltraktes.

In einer weiteren Ausführungsform können die Peptide zur Behandlung von chronischen Erkrankungen, teils vergesellschaftet mit den bereits erwähnten Erkrankungen eingesetzt werden, indem diese in geeigneter Form für die Behandlung benutzt werden.

Die Peptide können weiterhin eingesetzt werden zur Behandlung von Erkrankungen im akuten Stadium.

Vor diesem Hintergrund betrifft die vorliegende Beschreibung ferner eine pharmazeutische bzw. kosmetische Zusammensetzung, die als wirksamen Bestandteil ein Peptid in einer antimikrobiell wirksamen Menge, vorzugsweise im Bereich von 1- 50 µg/ml enthält.

In einer weiteren Ausführungsform können die Peptide zur Beschichtung von Materialien, vorzugsweise Katheter, Kontaktlinsen oder orthopädischer Implantate verwendet werden.

Durch die besonders hohe Wirksamkeit der Peptide gegenüber pathogenen Bodenkeimen sind die Peptide auch einsetzbar zum Schutz von Pflanzen, vorzugsweise durch Herstellung transgener Pflanzen durch Klonierung der für die Peptide kodierenden Nukleinsäuren.

Auch für den Einsatz zur Infektionsprophylaxe und der Behandlung von Infektionen bei Tieren, vorzugsweise Nutztiere und Haustiere, sind die Peptide geeignet.

Die Peptide können eingesetzt werden zur Diagnose oben bereits erwähnter Erkrankungen, indem beispielsweise Antikörper gegen eines oder mehrere der Peptide oder seiner Derivate oder ihrer Fragmente hergestellt werden und die Konzentration in Körperflüssigkeiten oder Geweben eines oder mehrerer Peptide über immunologische Verfahren gemessen wird.

Die IFPS-Peptide können als Arzneimittel zubereitet werden. Diese enthalten eines oder mehrere der IFPS-Peptide oder ein physiologisch verträgliches Salz dieser Peptide. Die Form und Zusammensetzung der Arzneimittel, welche eines oder mehrere der IFPS-Peptide enthalten, richtet sich nach der Art der Verabreichung. Die Arzneimittel eines oder mehrere der IFPS-Peptide enthaltend können topisch, parenteral, intranasal, oral oder mittels Inhalation verabreicht werden. Vorzugsweise werden diese Arzneimittel enthaltend eines oder mehrere der IFPS-Peptide in für den topischen Einsatz geeigneten Formulierungen, vorzugsweise Cremes, Salben oder Lösungen, oder mit einem Injektionspräparat, entweder als Lösung oder als Lyophilisat zur Auflösung unmittelbar vor Gebrauch konfektioniert. Die Arzneimittelzubereitungen können außerdem Hilfsstoffe enthalten, die abfülltechnisch bedingt sind, einen Beitrag zur Löslichkeit, Stabilität oder Sterilität des Arzneimittels leisten oder den Wirkungsgrad der Aufnahme in den Körper erhöhen.

Die Bestimmung der biologischen Aktivität für die IFPS-Peptide basiert auf Messungen gegen international gebräuchliche Referenzpräparationen für antibiotische Substanzen. Die IFPS-Peptide eignen sich besonders auch für die Langzeit-Therapie bei Infektionserkrankungen, da sie über eine ausgezeichnete biologische Wirksamkeit und Spezifität gegenüber Feuchtkeimen, insbesondere *Pseudomonas aeruginosa*, verfügen und andererseits als körpereigene Peptide auch bei Dauerbehandlung keine Immunreaktion auslösen.

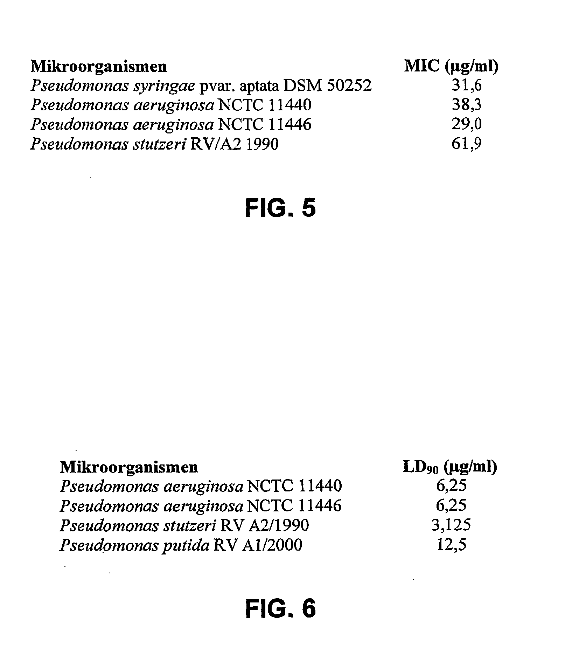

Zur Bestimmung der Aktivität wurden die IFPS-Peptide auf ihre antimikrobielle Wirkung hin getestet. Im Radial-Diffusions-Assay konnten die in Fig. 5 angegebenen Aktivitäten gegen verschiedene Bakterienstämme gemessen werden.

Die Isolierung der IFPS cDNA, ihr Vorkommen in verschiedenen Geweben, die Herstellung eines rekombinanten IFPS-Peptids sowie der Nachweis dessen antimikrobiellen Aktivität werden in folgenden Beispielen beschrieben.

### 1) Isolierung der IFPS cDNA und Bestimmung der genomischen Sequenz

Bei *in silico*-Analysen des humanen Genoms mithilfe des "Ensembl"-Servers wurde eine 82 Aminosäuren lange Sequenz bestehend aus den konservierten "EF-hand"-Domänen des Profilaggrins benutzt, um nach putativ neuen Mitgliedern der "S 100 Fused Type Protein" (SFTP)-Familie zu suchen. Unter anderem wurde eine Sequenz zwischen den bekannten Genen für Profilaggrin (*FLG*) und Cornulin (*CRNN* oder *C1orf10*) gefunden, worauf eine hypothetische cDNA-Sequenz durch Exon-Analysen der umgebenden genomischen Region mittels des "FuzzyFinder"-Programms erstellt wurde. Die untersuchte Region enthielt zwei putative Exons. Die gesamte cDNA-Sequenz wurde aus Keratinocyten-cDNA bestimmt. Hierzu wurde Gesamt-RNA aus humanen Vorhaut-Keratinocyten mit TRIzol^{®} nach Protokoll isoliert. Nach DNase-Verdau wurden 3 µg der Gesamt-RNA unter Verwendung des SMART RACE cDNA Amplification Kits (Clontech, Heidelberg) in cDNA umgeschrieben. Mittels 5'- und 3'-RACE ("Rapid Amplification of cDNA Ends") wurden die terminalen Bereiche der cDNA bestimmt; das fast 9 kb große Exon3 des IFPS wurde über die "Short-Range Overlapping PCR"-Methode amplifiziert und anschließend sequenziert. Die komplette cDNA-Sequenz ist unter der GenBank Zugangsnummer AY827490 beim "National Center for Biotechnology Information" (NCBI) verfügbar. Die IFPS cDNA-Sequenz ist insgesamt 9117 bp lang, besitzt einen offenen Leserahmen von 7176 bp und ein Polyadenylierungssignal (AATAAA) neun Nucleotide 5' vor Beginn der Polyadenylierung. Sie ist ähnlich der cDNA-Sequenz aller bisher bekannter "SFTP"s strukturiert mit insgesamt zwei Introns (1048 und 1089 bp) und drei Exons (52, 160 und 8907 bp), von denen nur die letzten beiden die proteinkodierende Sequenz tragen. Die abgeleitete Proteinsequenz besteht aus 2391 Aminosäuren und besitzt N-terminal eine S100- und EF-hand-Domäne, anschließend eine Spacer-Region gefolgt von zwei verschiedenen Multiple Tandem Repeats-Regionen (10xA und 14xB), die beide 75-77 Aminosäuren lang sind, sich aber in ihrer Sequenz unterscheiden und durch einen kurzen Spacer getrennt sind, und abschließend den C-Terminus.

### 2) Nachweis von IFPS in verschiedenen Gewebeproben

Zur Bestimmung der IFPS-mRNA-Expression wurde mit cDNA-Proben verschiedener humaner Gewebe und Zellen eine RT-PCR mit Intron-überspannenden Primern durchgeführt. Die IFPS-mRNA konnte dabei lediglich in der Haut und in kultivierten Vorhaut-Keratinocyten nachgewiesen werden. In allen anderen untersuchten Geweben wie Milz, Thymus, Dünndarm, Knochenmark, Tonsillen, Magen, Leber, Larynx, Pharynx, Dickdarm, Polypen, Lunge, Speicheldrüse, Niere, Uterus, Lymphknoten, Neutrophilen, bronchialen und trachealen Epithelzellen konnte keine mRNA gefunden werden. In kultivierten Keratinozyten wird die IFPSmRNA durch Zugabe von Ca²⁺ zum Medium stark hochreguliert, wodurch angenommen werden kann, dass IFPS ähnlich wie auch Profilaggrin und Involucrin als Marker für Keratinozyten-Differenzierung dienen könnte.

### 3) Konstruktion, Expression und Aufreinigung eines rekombinanten IFPS-Peptids

Ein Fragment des IFPS, das die letzte B-Repeat-Domäne und den kompletten C-Terminus einschließt (148 Aminosäuren), wurde rekombinant im SUMO-System (Invitrogen, Karlsruhe; LifeSensors, Malvern) hergestellt.

Hierzu wurde das C-terminale Fragment mittels PCR von Keratinocyten-cDNA mit einer *Pfu*Polymerase amplifiziert. Das amplifizierte PCR-Fragment wurde anschließend zusätzlich mit einer *Taq*-Polymerase inkubiert, um an den 3'-Enden des PCR-Produktes Desoxyadenosin-Überhänge zu generieren, mithilfe derer das Fragment in den pET SUMO-Vektor ligiert wurde. Der Vektor wurde in chemokompetente *E*. *coli* TOP10-Zellen transformiert und positive Klone wurden über Antibiotikaresistenz (hier Kanamycin) und Kolonie-PCR identifiziert. Aus Übernachtkulturen in LB-Medium und entsprechendem Antibiotikum (50 µg/ml) wurde der Vektor isoliert, sequenziert und bei korrekter Nucleotidsequenz in *E. coli* BL21(DE3)pLysS Zellen transformiert, welche zur Proteinexpression in der Lage sind. Positive Klone wurden wie zuvor identifiziert.

Die Expression des Fusionsproteins in BL21(DE3)pLysS erfolgte bei 37 °C und 200 rpm in LB Flüssigmedium mit 14 mM Glucose unter Zugabe von 34 µg/ml Chloramphenicol und 50 µg/ml Kanamycin. Die Kultur wurde bei einer Optischen Dichte (OD) bei einer Wellenlänge von 600 nm von 0,4-0,6 mit IPTG induziert und weitere drei Stunden inkubiert. Die Zellen wurden anschließend pelletiert, pro 100 ml Kultur in einem Milliliter Lysis-Äquilibrierungspuffer (LEW) aufgenommen und bei -80°C gelagert.

Der Aufschluss der Zellen erfolgte durch dreimaliges Auftauen und Einfrieren bei 30°C und - 80°C ("Freeze and Thaw"-Methode) und anschließende Ultraschall-Behandlung, wobei das Volumen pro 50 ml-Röhrchen zwischen 4 und 5 ml betrug. Die Ultraschall-Behandlung wurde auf Eis für 5 min durchgeführt jeweils in Zyklen mit 30 s Ultraschall (60/40-Intervall) und 15 sec Pause. Die Zellreste wurden bei 4 °C und 12000 x g für 45 min abzentrifugiert und der Überstand sterilfiltriert.

Die weitere Aufreinigung erfolgte über eine Nickel-Chelat-Säule (Protino® Ni-IDA 1000 packed columns, Macherey-Nagel, Düring). Hierbei wurde das Fusionsprotein durch Wechselwirkungen zwischen den Histidinen des zusätzlich exprimierten Tags und den an die Säulenmatrix gebundene Nickelionen nach Protokoll aufgereinigt.

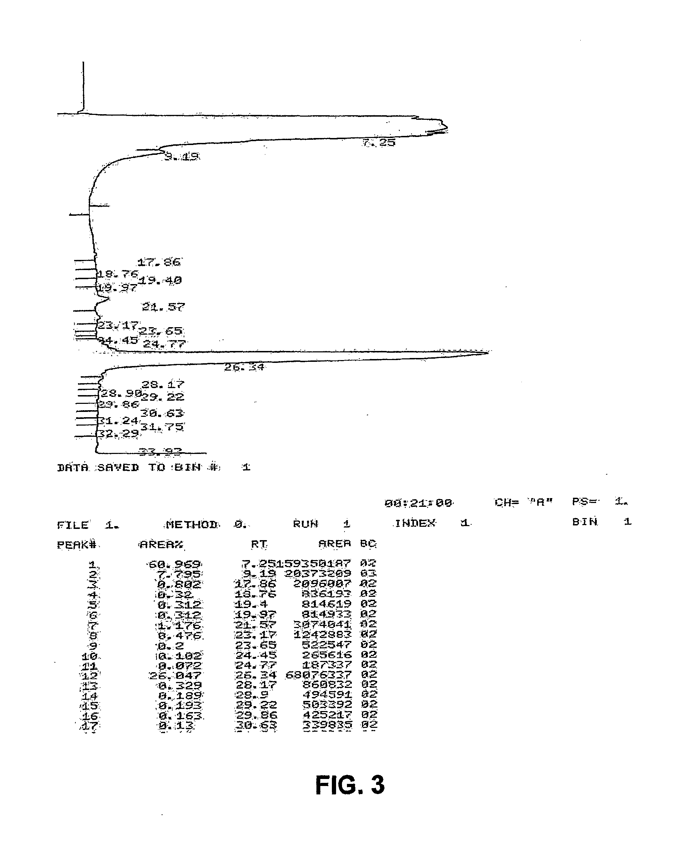

Um Verunreinigungen durch bakterielle Proteine weitestgehend auszuschließen, wurde das Fusionsprotein weiter über eine Umkehrphasen- ("Reversed Phase", RP) HPLC mit einer RP8-Säule und Vorsäule (SP 250/10 bzw. SP 50/10 NUCLEOSIL 300-7 C8, Macherey-Nagel, Düring) über einen linearen Gradienten gereinigt (Fig. 3). Puffer A war hierbei Wasser mit 0,1 % Trifluoressigsäure (TFA), Puffer B Acetonitril mit 0,1 % TFA und der Gradient wie folgt: 0-5 min 90 % A/ 10% B, bis 30 min linear auf 40 % A/ 60 % B, in 5 min linear auf 100 % B; die Flussgeschwindigkeit betrug 3 ml/min und die Detektion erfolgte bei 215 nm.

Die gewonnene Fraktion mit einer Retentionszeit von 26,34 min wurde aufgefangen und im ESI-Verfahren massenspektrometrisch analysiert, um die Größe des exprimierten Proteins zu überprüfen. Das Protein dieser Fraktion (IFPS₂₂₄₄₋₂₃₉₁-SUMO-Fusionsprotein) besaß die korrekte Größe von 29141,3 Da; die Konzentration wurde anhand des Absorptionskoeffizienten photometrisch bestimmt und die Fraktion wurde gefriergetrocknet.

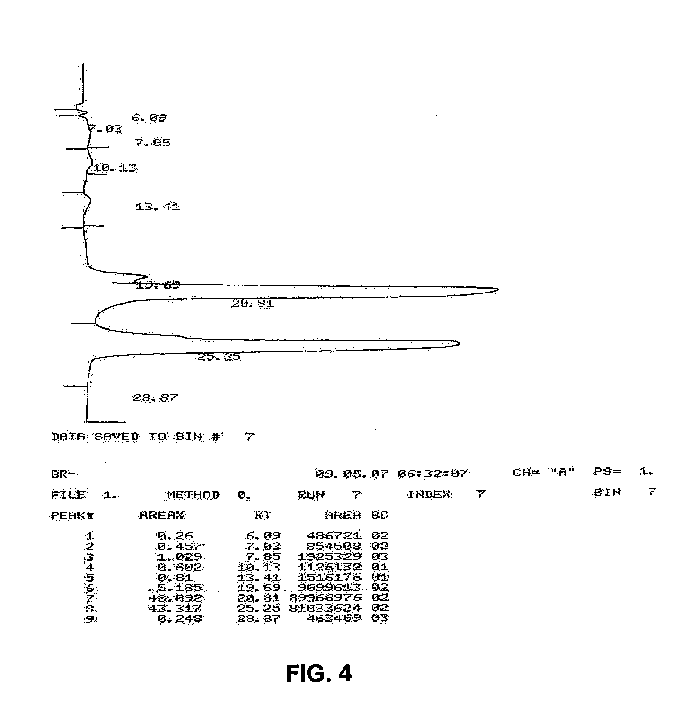

Zum Verdau wurden je 100 µg Fusionsprotein in 500 µl 1xPBS (Phosphate Buffered Saline) aufgenommen und mit 2,5 U SUMO Protease1 (LifeSensors, Malvern) über mindestens 3 h bei 30°C inkubiert. Die Abtrennung des Fusiontags und eventuell unverdauten Fusionsproteins erfolgte über RP-HPLC mit einer RP4-Säule (Bakerbond C4 5u 300A, 250 x 4,60 mm micron, Phenomenex, Aschaffenburg) (Fig. 4). Puffer A war hierbei Wasser mit 0,1 % Trifluoressigsäure (TFA), Puffer B Acetonitril mit 0,1 % TFA und der Gradient wie folgt: Anfangskonzentration 90 % A/ 10 % B, bis 10 min linear auf 60 % A/ 40 % B, in 15 min linear auf 30 % A/ 70 % B, in weiteren 10 min auf 100 % B; die Flussgeschwindigkeit betrug 0,5 ml/min und die Detektion erfolgte bei 215 nm.

Die gewonnene Fraktion mit einer Retentionszeit von 20,81 min (entspricht 55 % Acetonitril) wurde aufgefangen und im ESI-Verfahren massenspektrometrisch analysiert, um die Größe des exprimierten Proteins (IFPS₂₂₄₄₋₂₃₉₁) zu überprüfen. Das Protein dieser Fraktion besaß die korrekte Größe von 15874,5 Da; die Konzentration wurde anhand des Absorptionskoeffizienten photometrisch bestimmt und die Fraktion wurde für die Weiterverwendung gefriergetrocknet.

### 4) Nachweis antimikrobieller Aktivität im Radial-Diffusions-Hemmtest

Im Radial-Diffusions-Hemmtest wird die antimikrobielle Aktivität von Peptiden, beispielsweise ein C-terminales IFPS-Fragment, gegen in Agarose suspendierte Mikroorganismen, hier beispielsweise *Pseudomonas aeruginosa*, untersucht. Dazu werden zu untersuchende Proteine wie in Beispiel 3 aufgeführt aufgereinigt und lyophilisiert und in 0,01% Essigsäure aufgenommen.

Zum Test der antimikrobiellen Aktivität wird wie folgt verfahren: Aus einer logarithmisch wachsenden Kultur in TSB (Tryptic Soy Broth)-Medium (Sigma) werden 4 x 10⁶ KBE (Kolonie-bildende Einheiten) in eine 42 °C-warme Agarose-Lösung (1 % TSB, 10 mM Natriumphosphatpuffer pH 7,4, 1 % Agarose, 0,02 % Tween 20) gegeben. Nach Abkühlen der Agaroselösung in Petrischalen werden unter sterilen Bedingungen Kavitäten mit einem Durchmesser von 3 mm in die feste Agarose gestanzt.

In die Kavitäten werden je 5 µl einer zu testenden Peptid-Lösung gegeben und bei 37 °C über Nacht inkubiert. Als Negativkontrolle dient das Lösemittel 0,01 % Essigsäure, als Positivkontrolle humanes Lysozym (500 ng/ml, Sigma).

Am darauf folgenden Tag wird eine weitere 42°C warme nährstoffreiche Agaroselösung (1 % Agarose, 4 % Pepton, 0,5 % Glucose, 1 % Natriumchlorid, 0,5 % Kaliumhydrogenphosphat, pH 7,2) in die Petrischalen gegossen und weitere drei bis vier Stunden im Brutschrank inkubiert.

Zur Auswertung der antimikrobiellen Aktivität der Proben werden entstandene Hemmhöfe vermessen.

### 5) Nachweis der antimikrobiellen Aktivität gegen Pseudomonas aeruginosa im Mikro-Verdünnungs-Hemmtest

Die antimikrobielle Aktivität der IFPS-Fragmente wird zusätzlich in einem Flüssigkultur-Testsystem bestimmt. Dazu werden die verschiedenen *Pseudomonas aeruginosa-Stämme* in BHI (Brain Heart Infusion)-Medium kultiviert, in 10 mM Natriumphosphat-Puffer (pH 7,3) mit 1 % TBS verdünnt und davon 90 µl mit 10 µl der Peptid-Lösung (in unterschiedlichen Konzentrationen) für 2 h bei 37 °C inkubiert.

Nach der Inkubationszeit werden die Ansätze 1:10 und 1:100 in 10 mM Natriumphosphat-Puffer (pH 7,3) mit 1 % TBS verdünnt. Von diesen Verdünnungen werden jeweils 100 µl in drei Parallelansätzen auf BHI-Agar ausplattiert.

Nach Inkubation der Platten für 24 h bei 37 °C werden die kolonie-bildenden Einheiten (KBE) ausgezählt. Als Kontrolle wird je ein Ansatz nur mit 10 mM Natriumphosphat-Puffer direkt vor und nach der zweistündigen Inkubation ausplattiert. 6) Antimikrobielle Aktivität von C-terminalen IFPS-Peptiden gegen *Pseudomonas ssp.*

Die antimikrobielle Aktivität des IFPS-Peptids wurde unter Anwendung sowohl des Radial-Diffusions- und des Mikro-Dilutions-Hemmtests gegen verschiedene Mikroorganismen untersucht.

Die Minimale Hemmhofkonzentration (MIC) bezeichnet die Peptidkonzentration, die minimal erforderlich ist, um das Wachstum von einigen *Pseudomonas ssp* nach Inkubationszeit (s. Beispiel 5) vollständig zu hemmen. Für das C-terminale IFPS-Peptid 2 wurde beispielhaft die in Fig. 5 angegebene minimale Hemmhofkonzentrationen gefunden, wobei die Konzentration sich auf das in die Kavität eingefüllte Volumen von 5 µl bezieht.

Die Letale Dosis, bei der 90 % der Mikroorganismen abgetötet werden (LD₉₀) lässt sich aus dem reduzierten Auswachsen der KBE im Mikro-Dilutions-Hemmtest bestimmen. Die LD₉₀ für einige *Pseudomonas ssp* sind in Fig. 6 angegeben.

## Patentansprüche

1. Verwendung eines Peptids mit der in SEQ ID:NO 1, SEQ ID:NO 2 oder SEQ ID:NO 3 dargestellten Sequenz zur Herstellung eines antimikrobiell wirkenden Arzneimittels zur Behandlung von Infektionen durch Bakterien der Gattung *Pseudomonas.*

2. Verwendung nach Anspruch 1 zur Herstellung eines Arzneimittels zur Behandlung von infektiösen Erkrankungen des menschlichen oder tierischen Epithelgewebes.

3. Verwendung nach einem der vorhergehenden Ansprüche zur Behandlung von infektiösen Erkrankungen des menschlichen Organismus mit Beteiligung der Haut, des Magen-Darm-Traktes, der Atemwege oder des Urogenitalapparates

4. Verwendung nach einem der vorhergehenden Ansprüche zur Herstellung eines Arzneimittels für die Substitutionstherapie.

5. Verwendung nach einem der vorhergehenden Ansprüche zur Behandlung von chronischen Infektionen durch Bakterien der Gattung *Pseudomonas.*

6. Verwendung nach einem der vorhergehenden Ansprüche zur Behandlung von Infektionen mit *Pseudomonas aeruginosa.*

7. Verwendung nach einem der vorhergehenden Ansprüche, **dadurch gekennzeichnet, dass** in SEQ ID:NO 1 Position 96 (G) gegen D und/oder in Position 98 (S) gegen D, G, N oder Q, in Position 100 (V) gegen G oder A, in Position 102 (K) gegen R oder T, in Position 108 (P) gegen S oder N oder R in Position 112 (D) gegen E oder N oder Y, in Position 115 (H) gegen Q oder E oder K, in Position 116 (T) gegen S, in Position 118 (Y) gegen F, in Position 123 (G) gegen R oder D oder A, in Position 124 (S) gegen R oder L und/oder in Position 125 (R) gegen Q oder R ausgetauscht ist.

8. Medizinisches Instrument, Katheter, medizinisches Implantat oder Kontaktlinse **gekennzeichnet durch** eine ein Peptid mit der in SEQ ID:NO 1, SEQ ID:NO 2 oder SEQ ID:NO 3 dargestellten Sequenz aufweisenden Beschichtung.

9. Kosmetische Zusammensetzung, **gekennzeichnet durch** ein Peptid mit der in SEQ ID:NO 1, SEQ ID:NO 2 oder SEQ ID:NO 3 dargestellten Sequenz.

10. Verwendung nach einem der Ansprüche 1 bis 7 oder kosmetische Zusammensetzung nach Anspruch 9, **dadurch gekennzeichnet, dass** die Konzentration des Peptids im Arzneimittel bzw. in der kosmetischen Zusammensetzung 1 bis 50 µg / ml beträgt.

## Claims

1. Use of a peptide having the sequence shown in SEQ ID NO.: 1, SEQ ID NO.: 2 or SEQ ID NO.: 3 for the preparation of an antimicrobial active drug for the treatment of infections caused by bacteria of the genus Pseudomonas.

2. The use according to claim 1 for the preparation of a drug for the treatment of infectious diseases of the human or animal epithelial tissue.

3. The use according to any one of the preceding claims for the treatment of infectious diseases of the human organism involving the skin, the gastrointestinal tract, the respiratory tract, or the urogenital system.

4. The use according to any one of the preceding claims for the preparation of a drug for substitution therapy.

5. The use according to any one of the preceding claims for the treatment of chronic infections caused by bacteria of the genus Pseudomonas.

6. The use according to any one of the preceding claims for the treatment of infections caused by Pseudomonas aeruginosa.

7. The use according to any one of the preceding claims, **characterized in that** in SEQ ID NO.: 1 position 96 (G) is substituted with D and/or in position 98 (S) with D, G, N or Q, in position 100 (V) with G or A, in position 102 (K) with R or T, in position 108 (P) with S or N or R, in position 112 (D) with E or N or Y, in position 115 (H) with Q or E or K, in position 116 (T) with S, in position 118 (Y) with F, in position 123 (G) with R or D or A, in position 124 (S) with R or L and/or in position 125 (R) with Q or R.

8. A medical instrument, catheter, medical implant, or contact lens **characterized by** a coating having a peptide with the sequence shown in SEQ ID NO.: 1, SEQ ID NO.: 2, or SEQ ID NO.: 3.

9. A cosmetic composition **characterized by** a peptide having the sequence shown in SEQ ID NO.: 1, SEQ ID NO.: 2 or SEQ ID NO.: 3.

10. The use according to any one of claims 1 to 7 or the cosmetic composition according to claim 9, **characterized in that** the concentration of the peptide in the drug and the cosmetic composition, respectively, is 1 to 50 µg/ml.

## Revendications

1. Utilisation d'un peptide ayant une séquence telle que représentée dans SEQ ID NO. : 1, SEQ ID NO. : 2 ou SEQ ID NO. : 3 pour la préparation d'un médicament actif antimicrobien pour le traitement d'infections causées par des bactéries du genre Pseudomonas.

2. L'utilisation conformément à la revendication 1 pour la préparation d'un médicament pour le traitement des maladies infectieuses du tissu épithélial humain ou animal.

3. L'utilisation conformément à n'importe laquelle des revendications précédentes pour le traitement des maladies infectieuses de l'organisme humain impliquant la peau, les voies gastro-intestinales, les voies respiratoires ou l'appareil urogénital.

4. L'utilisation, conformément à n'importe laquelle des revendications précédentes pour la préparation d'un médicament pour thérapie de substitution.

5. L'utilisation, conformément à n'importe laquelle des revendications précédentes pour le traitement d'infections chroniques causées par des bactéries du genre Pseudomonas.

6. L'utilisation, conformément à n'importe laquelle des revendications précédentes pour le traitement d'infections causées par des bactéries du genre Pseudomonas aeruginosa.

7. L'utilisation conformément à n'importe laquelle des revendications précédentes, **caractérisée par le fait que** dans SEQ ID NO. : 1 la position 96 (G) est substituée par D et/ou la position 98 (S) est substituée par D, G, N ou Q, la position 100 (V) par G ou A, la position 102 (K) par R ou T, la position 108 (P) par S ou N ou R, la position 112 (D) par E ou N ou Y, la position 115 (H) par Q ou E ou K, la position 116 (T) par S, la position 118 (Y) par F, la position 123 (G) par R ou D ou A, la position 124 (S) par R ou L et ou la position 125 (R) par Q ou R.

8. Un instrument médical, cathéter, implant médical ou lentille de contact **caractérisé par** un revêtement ayant un peptide avec la séquence représentée dans la SEQ ID NO. : 1, SEQ ID NO. : 2 ou SEQ ID NO. : 3.

9. Une composition cosmétique **caractérisée par** un peptide ayant une séquence telle que représentée dans SEQ ID NO. : 1, SEQ ID NO. : 2 ou SEQ ID NO. : 3.

10. L'utilisation conformément à n'importe laquelle des revendications 1 à 7 pour la composition cosmétique conformément à la revendication 9, **caractérisée par le fait que** la concentration de peptide dans le médicament et dans la composition cosmétique, respectivement, sont comprises entre 1 et 50µg/ml.


### SEQUENZPROTOKOLL

<110> Christian-Albrechts-Universität zu Kiel
<120> C-terminale Ifapsoriasinfragmente als antimikrobielle Peptide, deren Herstellung und Verwendung
<130> P 5681
<160> 3
<170> PatentIn version 3.3
<210> 1
   <211> 148
   <212> PRT
   <213> Homo sapiens
<400> 1
   Gly Ser Arg Thr Thr Gly Arg Arg Gly Ser Gly His Ser Glu Ser Ser
   1               5                   10                  15
   Asp Ser Glu Val His Ser Trp Gly Ser His Thr His Ser Gly His Ile
               20                  25                  30
   Gln Gly Gln Ala Gly Ser Gln Gln Arg Gln Pro Gly Ser Thr Val His
           35                  40                  45
   Gly Arg Leu Glu Thr Thr His Gly Gln Thr Gly Asp Thr Thr Arg His
       50                  55                  60
   Gly His Ser Gly Tyr Gly Gln Ser Thr Gln Thr Gly Ser Arg Ser Ser
   65                  70                  75                  80
   Arg Ala Ser His Phe Gln Ser His Ser Ser Glu Arg Gln Arg His Gly
                   85                  90                  95
   Ser Ser Gln Val Trp Lys His Gly Ser Tyr Gly Pro Ala Glu Tyr Asp
               100                 105                 110
   Tyr Gly His Thr Gly Tyr Gly Pro Ser Gly Gly Ser Arg Lys Ser Ile
           115                 120                 125
   Ser Asn Ser His Leu Ser Trp Ser Thr Asp Ser Thr Ala Asn Lys Gln
       130                 135                 140
   Leu Ser Arg His
   145
<210> 2
   <211> 70
   <212> PRT
   <213> Homo sapiens
<400> 2
   Ser Ser Arg Ala Ser His Phe Gln Ser His Ser Ser Glu Arg Gln Arg
   1               5                   10                  15
   His Gly Ser Ser Gln Val Trp Lys His Gly Ser Tyr Gly Pro Ala Glu
               20                  25                  30
   Tyr Asp Tyr Gly His Thr Gly Tyr Gly Pro Ser Gly Gly Ser Arg Lys
           35                  40                  45
   Ser Ile Ser Asn Ser His Leu Ser Trp Ser Thr Asp Ser Thr Ala Asn
       50                  55                  60
   Lys Gln Leu Ser Arg His
   65                  70
<210> 3
   <211> 55
   <212> PRT
   <213> Homo sapiens
<400> 3
   Ser Ser Arg Ala Ser His Phe Gln Ser His Ser Ser Glu Arg Gln Arg
   1               5                   10                  15
   His Gly Ser Ser Gln Val Trp Lys His Gly Ser Tyr Gly Pro Ala Glu
               20                  25                  30
   Tyr Asp Tyr Gly His Thr Gly Tyr Gly Pro Ser Gly Gly Ser Arg Lys
           35                  40                  45
   Ser Ile Ser Asn Ser His Leu
       50                  55